(19) Europäisches Patentamt
European Patent Office
Office européen des brevets

(11) Veröffentlichungsnummer : **0 495 339 A2**

# (12) EUROPÄISCHE PATENTANMELDUNG

(21) Anmeldenummer : **91810963.8**

(22) Anmeldetag : **11.12.91**

(51) Int. Cl.[5] : **C07D 303/24,** C07D 303/22, C07D 301/28

(30) Priorität : **18.12.90 CH 4012/90**

(43) Veröffentlichungstag der Anmeldung : **22.07.92 Patentblatt 92/30**

(84) Benannte Vertragsstaaten : **DE ES FR GB IT NL**

(71) Anmelder : **CIBA-GEIGY AG**
**Klybeckstrasse 141**
**CH-4002 Basel (CH)**

(72) Erfinder : **Roth, Martin, Dr.**
**Oberdorf**
**CH-1735 Giffers (CH)**
Erfinder : **Wolleb, Heinz, Dr.**
**Chesalles**
**CH-1723 Marly (CH)**
Erfinder : **Truffer, Marc-André, Dr.**
**Les Plumasses**
**CH-1867 Antagnes/Ollon (CH)**

## (54) Verbessertes Verfahren zur Herstellung von Glycidylethern.

(57) Verbessertes Verfahren zur Herstellung von Glycidylethern der Formel I

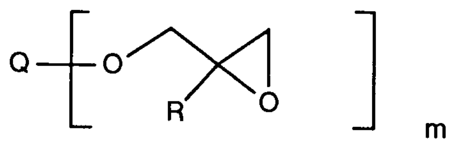

(I),

worin Q für einen m-wertigen aliphatischen, cycloaliphatischen oder araliphatischen Rest und R für -H oder $-CH_3$ stehen, und m eine ganze Zahl von 1 bis 10 ist, indem ein Alkohol der Formel $Q-(OH)_m$ mit m Mol eines Epihalohydrins der Formel II

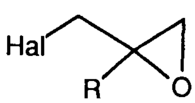

(II)

in Gegenwart eines Katalysators zum entspechenden Halohydrinether umgesetzt wird und der Halohydrinether mit einem Alkalimetallhydroxid zu einer Verbindung der Formel I dehydrohalogeniert wird, wobei Q, m und R die oben angegebene Bedeutung haben und Hal für Halogen steht, dadurch gekennzeichnet, dass man als Katalysator

a) Zinndifluorid oder
b) ein zweiwertiges Zinnhalogenid in Kombination mit einem Co-Katalysator verwendet.

EP 0 495 339 A2

Die vorliegende Erfindung betrifft ein verbessertes Verfahren zur Herstellung von aliphatischen, cycloaliphatischen oder araliphatischen Glycidylethern durch Umsetzung eines Alkohols und eines Epihalohydrins in Gegenwart bestimmter Katalysatoren und anschliessendes Dehydrohalogenieren des resultierenden Halohydrinethers mit einem Alkalimetallhydroxid.

Glycidylether können durch Umsetzung von Alkoholen und Epihalohydrinen hergestellt werden. Dabei werden verschiedenartige Katalysatoren, wie z.B. saure, basische oder Phasentransfer-Katalysatoren, eingesetzt. Als saure Katalysatoren werden sogenannte Lewis-Säuren, wie $AlCl_3$, $SbCl_5$, $SnCl_4$, $FeCl_3$, $ZnCl_2$ und $BF_3$, verwendet, wie z.B. in den US-Patenten Nr. 2538072, Nr. 3425961 und Nr. 4549008, und in den GB-Patenten Nr. 827450 und 982151 beschrieben. Die dabei bevorzugt eingesetzten Lewis-Säuren sind Bortrifluorid und insbesondere Zinn(IV)-tetrachlorid. Schliesslich wird im US-Patent Nr. 3686358 die Verwendung von zweiwertigem Zinnchlorid für die Herstellung von Dibromneopentylglykoldiglycidylether vorgeschlagen.

Es wurde nun gefunden, dass die Umsetzung von aliphatischen, cycloaliphatischen oder araliphatischen Alkoholen mit Epihalohydrinen unter Verwendung bestimmter Katalysatorsysteme auf der Basis von zweiwertigen Zinnhalogeniden viel selektiver verläuft als mit Bortrifluorid oder Zinntetrachlorid und zu Glycidylethern mit niedrigeren Chlor- und höheren Epoxidwerten führt.

Gegenstand der vorliegenden Erfindung ist daher ein verbessertes Verfahren zur Herstellung von Glycidylethern der Formel I

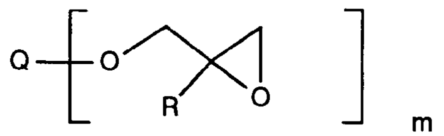

(I),

worin Q für einen m-wertigen aliphatischen, cycloaliphatischen oder araliphatischen Rest und R für -H oder $-CH_3$ stehen, und m eine ganze Zahl von 1 bis 10 ist,indem ein Alkohol der Formel $Q-(OH)_m$ mit m Mol eines Epihalohydrins der Formel II

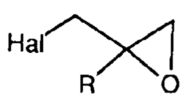

(II)

in Gegenwart eines Katalysators zum entspechenden Halohydrinether umgesetzt wird und der Halohydrinether mit einem Alkalimetallhydroxid zu einer Verbindung der Formel I dehydrohalogeniert wird, wobei Q, m und R die oben angegebene Bedeutung haben und Hal für Halogen steht, dadurch gekennzeichnet, dass man als Katalysator

a) Zinndifluorid oder

b) ein zweiwertiges Zinnhalogenid in Kombination mit einem Co-Katalysator verwendet.

Q als aliphatischer Rest kann geradkettig oder verzweigt, gesättigt oder ungesättigt sein und gegebenenfalls durch ein oder mehrere Sauerstoff- oder Schwefelatome in der Kette unterbrochen sein oder eine oder mehrere Ketogruppen enthalten.

Der cycloaliphatische Rest kann gesättigt oder ungesättigt sein und ein oder mehrere Ringsysteme aufweisen und gegebenenfalls eine Ketogruppe enthalten, wobei die Ringe durch Alkylgruppen substituiert und/oder überbrückt sein können.

Der aromatische Rest im araliphatischen Rest kann einen oder mehrere, gegebenenfalls ankondensierte Ringe aufweisen, wie z.B. Phenyl, Naphthyl, 4,4′-Diphenyl-, 4,4′-Diphenylmethan-, 4,4′-Diphenyl-(dimethyl)methan-, 4,4′-Diphenyloxyd-, -keton- oder -sulfon-Reste, welche ihrerseits durch $C_1$-$C_6$Alkyl, $C_1$-$C_6$Alkoxy oder Halogen substituiert sein können. Araliphatische Reste sind insbesondere Aralkylreste, in denen das Alkyl unverzweigt oder verzweigt sein kann und vorzugsweise 1 bis 3 C-Atome aufweisen.

$C_1$-$C_6$Alkyl und $C_1$-$C_6$Alkoxy können geradkettig oder verzweigt sein und bedeuten z.B. Methyl, Ethyl, Propyl, Isopropyl, Butyl, sek.-Butyl, tert.-Butyl, Pentyl und Hexyl bzw. Methoxy, Ethoxy, Propoxy, Isopropoxy, Butoxy, tert.-Butoxy, Pentoxy und Hexyloxy. Halogen kann Chlor, Brom oder Iod sein, bevorzugt aber ist es Brom und insbesondere Chlor.

Q kann gegebenenfalls mit funktionellen Gruppen substituiert sein, vorausgesetzt, dass sie den eingesetzten Katalysator nicht inhibieren und keine Nebenreaktionen mit den Epihalohydrinen eingehen.

Q als Alkylrest (bei m=1) bedeutet z.B. $C_1$-$C_{36}$Alkyl, bevorzugt $C_3$-$C_{26}$Alkyl. Beispiele für solche Reste sind Methyl, Ethyl, n-Propyl, Isopropyl, n-Butyl, sek.-Butyl, tert.-Butyl, Pentyl, Hexyl, Heptyl, Octyl, 2-Ethylhexyl, Nonyl, Decyl, Dodecyl, Tetradecyl, Hexadecyl, Octadecyl, Eicosyl, Docosyl, Tetracosyl und Pentacosyl.

Cycloalkylreste sind beispielsweise Cyclopentyl, Cyclohexyl, Cycloheptyl, Cyclooctyl und Cyclononyl, insbesondere aber Cyclohexyl.

Beispiele für mehrfunktionelle Alkohole der Formel Q-$(OH)_m$ sind aliphatische, cycloaliphatische oder araliphatische Strukturelemente, wie z.B. Butandiole, Trimethylolpropan, Bis-trimethylolpropan, Pentaerythrit, Cyclohexandiole, Tricyclodecandimethylol (z.B. Tricyclo[5.2.1.$0^{2.6}$]decan-4,8-dimethanol) oder Perhydrobisphenol-A.

Beispiele von Alkoholen der Formel Q-$(OH)_m$ sind:

Methanol, Ethanol, Propanol, Isopropanol, Butanol, sek.-Butanol, tert.-Butanol, Pentanol, tert.-Pentanol, Cyclopentanol, Hexanol, Cyclohexanol, Heptanol, Octanol, Decanol, Dodecanol, Tetradecanol, Pentadecanol, Hexadecanol, Heptadecanol, Octadecanol, Benzylalkohol, Diphenylmethanol, Ethylenglykol, Propan-1,2-diol, Propan-1,3-diol, Butan-1,4-diol, Butan-1,2-diol, Butan-2,3-diol, Diethylenglykol, Triethylenglykol, Pentan-1,5-diol, Hexan-1,6-diol, Hexan-2,4,6-triol, 2,2-Dimethyl-1,3-propandiol, 2-Ethyl-2-butylpropan-1,2-diol, 1,12-Dihydroxyoctadecan, Glycerin, Erythrit, Pentaerythrit, Sorbit, Mannit, Inosit, 1,1,1-Trimethylolpropan, 1,4-Dimethylolbenzol, 4,4′-Dimethyloldiphenyl, Dimethylolxylole, Dimethylolnaphthaline, Polyetheralkohole, wie Diglycerin, ferner Bis-(2,3-dihydroxypropylether), Triglycerin, Dipentaerythritol, Dimethylolanisole, beta-Hydroxyethylether von Polyalkoholen oder Phenolen, wie Diethylenglykol, Polyethylenglykol oder Hydrochinon-bis-(beta-hydroxyethylether), Bis-(beta-hydroxyethylether) von Bisphenolen, wie von 4,4′-Dihydroxydiphenyldimethylmethan, ferner beta-Hydroxyethylether von Glycerin, Pentaerythrit, Sorbit oder Mannit, Kondensate von Alkylenoxyden, wie Ethylen-, Propylen-, Butylen- oder Isobutylenoxyd, mit den oben genannten Polyalkoholen, ferner Hydroxyester, wie z.B. Monoglyceride, wie Monostearin, ferner Ethylenglykoldilactat, Monoester von Pentaerythrit, wie das Monoacetat, ferner halogenierte Alkohole, wie Glycerinmonochlorhydrin, 1,4-Dichlor-2,3-dihydroxybutan,, Pentaerythritmonochloride oder Dibromneopentylglykol, Bis-(4-hydroxycyclohexyl)-methan, Bis-(4-hydroxycyclohexyl)-dimethylmethan, 2,2-Bis-(4-hydroxycyclohexyl)-propan, Cyclohexan-1,1-dimethylol, 2,2,6,6-Tetramethylol-cyclohexanol, 2,2,5,5-Tetramethylol-cyclopentanol, 4-Methyl-2,2,6,6-tetramethylol-cyclohexanol, 2,2,6,6-Tetramethylol-cyclohexanon-4, 1,2-, 1,3- und 1,4-Dihydroxycyclohexan, 1,3-Dihydroxycyclopentan, 4,4′-Dihydroxydicyclohexyl, ferner Mercaptoalkohole, wie 2-Mercaptoethanol, alpha-Monothioglycerin, 2,2′,3,3′-Tetrahydroxydipropylsulfid oder 2,2′-Dihydroxydiethylsulfid.

Als Epihalohydrin kommen z.B. Epibromhydrin und insbesondere Epichlorhydrin in Frage.

m ist bevorzugt eine ganze Zahl von 1 bis 6.

Erfindungsgemäss bevorzugt in der Formel Q-$(OH)_m$ ist Q ein gesättigter aliphatischer oder cycloaliphatischer Rest.

Erfindungsgemäss bevorzugter gesättigter aliphatischer Rest in der Definition von Q ist Alkyl.

Erfindungsgemäss ganz bevorzugte Verbindungen der Formel Q-$(OH)_m$ sind primäre oder sekundäre monofunktionelle (m=1) oder mehrfunktionelle Alkohole, bei welchen m eine Zahl von 2 bis 6 darstellt.
Ebenfalls ganz bevorzugt ist Q ein mehrfunktioneller Rest mit bis zu 30 C-Atomen, wobei m eine ganze Zahl von 2 bis 6 ist.
Als zweiwertige Zinnhalogenide eignen sich Zinndichlorid, Zinndibromid und insbesondere Zinndifluorid. Diese Katalysatoren können, ohne die Umsetzung ungünstig zu beeinflussen, in einem weiten Konzentrationsbereich verwendet werden, jedoch ist eine Konzentration von 0,001 bis 0,5 Mol des Katalysators auf 1 Mol Alkohol der Formel Q-$(OH)_m$ vorteilhaft.
Erfindungsgemäss besonders geeignet als Katalysator ist Zinndifluorid allein.

Als Co-Katalysatoren können z.B. Kronenether oder Poly(alkylenoxid)-dialkylether verwendet werden. Sie werden zweckmässig in molaren Mengenverhältnissen zu den Zinnhalogeniden eingesetzt.
Beispiele für Kronenether sind z.B. 18-Crown-6, 15-Crown-5, 12-Crown-4, Benzo-18-crown-6, Benzo-15-crown-5, Dibenzo-18-crown-6, Dibenzo-24-crown 8 und Dicyclohexano-18-crown-6. Beispiele für Poly(alkylenoxid)-dialkylether sind z.B. Polyethylenglykol-400-dimethylether, Polyethylenglykol-500-dimethylether und Polyethylenglykol-1000-dimethylether.

Die Umsetzung der Verbindungen der Formel Q-$(OH)_m$ mit den Epihalohydrinen zu den Halohydrinethern erfolgt zweckmässig ohne Lösungsmittel, sie kann aber auch in Gegenwart eines inerten organischen Lösungsmittels durchgeführt werden. Geeignete Lösungsmittel sind z.B. halogenierte Kohlenwasserstoffe, wie Methylenchlorid, Chloroform, 1,2-Dichlorethan, 1,1,1-Trichlorethan, 1,2,2-Trichlorethylen, 1,4-Dichlorpropan und Chlorbenzol, ferner Toluol, Xylol, Hexan, Cyclohexan, Heptan, Octan, ferner Ester, wie Ethylacetat und Butylacetat, Ether, wie Diethyl- und Diisopropylether, Dioxan und Tetrahydrofuran. Auch Lösungsmittelgemische obiger Lösungsmittel in beliebigen Mengenverhätnissen können verwendet werden. Die Menge des Lösungsmittels bzw. des Lösungsmittelgemisches kann beliebig variieren. Sie wird zweckmässig so gewählt, dass die

Reaktandenkonzentrationen z.B. zwischen 10 und 70 Gew.% liegen.

Das Epihalohydrin wird zweckmässig zwischen 0,8 und 1,3 Mol Epihalohydrin pro Hydroxylgruppe gemäss der Formel Q-$(OH)_m$, bevorzugt aber in stöchiometrischen Mengen oder in einem leichten Ueberschuss, z.B. bis zu 10% Ueberschuss, bezogen auf die molare Epihalohydrinmenge, eingesetzt.

Man führt die Umsetzung des Alkohols mit dem Epihalohydrin z.B. zwischen 0 °C und der Rückflusstemperatur des Reaktionsgemisches, vorzugsweise zwischen 80 und 150 °C, durch. Die Gesamtreaktionszeiten variieren je nach Alkohol zwischen 30 Min. und 24 Stunden.

Das Anlagerungsprodukt (Halohydrinether) zwischen dem Epihalohydrin und dem Alkohol wird zweckmässig nicht isoliert und direkt dehydrohalogeniert.

Bei der Durchführung des erfindungsgemässen Verfahrens ist die Reihenfolge der Zugabe der Komponenten nicht wesentlich. Mögliche Verfahrensvarianten sind z.B.:

Der Alkohol wird zusammen mit dem Katalysator vorgelegt und auf die benötigte Reaktionstemperatur aufgeheizt. Das Epihalohydrin wird dann innerhalb geeigneter Zeitperioden zudosiert, so dass die Temperatur innerhalb eines geeigneten Bereiches bleibt. Dann lässt man solange weiterreagieren, bis die analytische Untersuchung des Reaktionsgemisches (z.B. mittels Gaschromatographie, Epoxidtitration, Chlorbestimmung oder HPLC) den gewünschten Umsatz zum entsprechenden Halohydrinether anzeigt. Alle Komponenten, d.h. der Alkohol, das Epihalohydrin und der Katalysator, werden vorgelegt, und auf die benötigte Reaktionstemperatur aufgeheizt und bis zum gewünschten Umsatz an Epihalohydrinether weiterreagiert.

Die weiteren Verfahrensschritte sind bei allen obigen Verfahrensvarianten gleich:

Ein eventuell vorhandener Ueberschuss an Epihalohydrin kann vor dem Dehydrohalogenieren destillativ aus dem Reaktionsgemisch entfernt werden. Ebenfalls lässt sich auf dieser Stufe ein grosser Teil des Zinnhalogenids bzw. des Katalysatorsystems durch Filtration abtrennen und damit wieder verwerten bzw. einsetzen. Die derart zurückgewonnene Menge an Katalysator ist dabei abhängig von der Löslichkeit im Reaktionsmedium, kann aber z.B. bei Zinndifluorid in günstigen Fällen bis mehr als 90% betragen. Durch Zugabe eines inerten organischen Lösungsmittels lässt sich gegebenenfalls die Ausbeute an zurückgewonnenem Katalysator erhöhen.

Die Dehydrohalogenierung des Halohydrinethers zum Glycidylether kann nach den bekannten, beim Arbeiten mit Zinntetrachlorid- oder Bortrifluorid-Katalysatoren üblichen Methoden mit einem Alkalimetallhydroxid als Base unter Abspaltung von Alkalimetallhalogenid erfolgen. Die theoretisch erforderliche stöchiometrische Menge kann dabei gegebenenfalls auch über- oder unterschritten werden, um z.B. die optimalen niedrigen Chlorwerte des Endprodukts zu erreichen. Generell sind Mengen von 0,80 bis 1,30 Mol Alkalimetallhydroxid pro Hydroxylgruppe und Temperaturen zwischen 30 und 60°C bevorzugt. Der Einsatz eines inerten organischen Lösungsmittels ist z.B. angezeigt, wenn die Viskosität der Reaktionsmischung zu hoch ist.

In manchen Fällen kann die Glycidylisierung vorteilhafterweise unter azeotroper Entfernung des Wassers in Gegenwart von Alkalimetallhydroxid durchgeführt werden. Das Alkalimetallhydroxid wird während der azeotropen Entwässerung vorzugsweise unter vermindertem Druck zugesetzt. Wasser, das als Lösungsmittel für das Alkalimetallhydroxid dient, und solches, das bei der Reaktion gebildet wird, wird auf diese Weise laufend aus dem Reaktionsgemisch entfernt.

Das bei der Reaktion gebildete Salz, wie z.B. Kochsalz, kann entweder ausgewaschen, abfiltriert oder abzentrifugiert werden. Ueberschüssiges Epihalohydrin wird gegebenenfalls im Vakuum abdestilliert.

Die erfindungsgemäss als Ausgangsprodukte eingesetzten Verbindungen stellen bekannte Produkte dar und sind z.T. auch im Handel erhältlich.

Das vorliegende verbesserte Verfahren weist gegenüber vorbekannten Verfahren mehrere Vorteile auf, wie eine bessere Selektivität, was zu niedrigeren Chlor- und höheren Epoxidwerten führt, ferner eine bessere Prozess-Sicherheit, da die eingesetzten zweiwertigen Zinnhalogenide bzw. Katalysatorsysteme die Epihalohydrine nicht polymerisieren, sowie besonders bei Verwendung von Zinndifluorid eine bessere Rückgewinnung des Zinnsalzes durch einfache Filtration, da Zinndifluorid in organischen Lösungsmitteln schwer löslich ist. Ausserdem kann die erste Reaktionsstufe oft in der Schmelze ohne Lösungsmittel durchgeführt werden.

Ferner fallen die erfindungsgemäss hergestellten Produkte in hoher Reinheit an. Bei Verwendung von Zinndifluorid ist das Resultat der vorliegenden Erfindung umso überraschender als Zinndifluorid keine eigentliche Lewis-Säure ist.

Die erfindungsgemäss hergestellten Glycidylether werden häufig in Epoxidharz-Formulierungen zur Modifikation bestimmter Eigenschaften eingesetzt, z.B. als Reaktivverdünner, Flexibilisatoren, Adhäsionsverbesserer, u.s.w.. Diese Formulierungen enthalten gegebenenfalls zusätzliche andere Epoxidharze, wie z.B. Bisphenol-A-epoxidharze oder Epoxidnovolacke, und übliche Härtungsmittel, wie z.B. Amine, Anhydride, Phenole oder katalytische Härter. Die Formulierungen finden Anwendung in verschiedensten Applkationsgebieten z.B. als Lackharze, Tauchharze, Imprägnierharze, Klebstoffe, Dichtungsmittel, Enkapsuliermassen und Isoliermaterialen. Die erfindungsgemäss erhaltenen Glycidylether weisen aufgrund ihrer aliphatischen Struktur ausgezeichnete Aussenbewitterungsbeständigkeit auf.

Die folgenden Beispiele beschreiben die Erfindung näher. Wenn nichts anderes vermerkt ist, bedeuten Teile Gewichtsteile.

Beispiel 1 (Butan-1,4-dioldiglycidylether):

In ein Reaktionsgefäss, versehen mit Rührer, Rückflusskühler, Tropftrichter und Thermometer, gibt man 180,24 g (2,0 Mol) 1,4-Butandiol und 6,27 g (0,04 Mol) Zinndifluorid und erwärmt auf 130°C Innentemperatur. Dann werden unter gutem Rühren 388,6 g (4,20 Mol) Epichlorhydrin innert 2 Stunden bei einer Temperatur von 130 bis 140°C zudosiert, wobei die Reaktion am Anfang exotherm ist. Man lässt 3 Stunden bei dieser Temperatur nachreagieren, kühlt auf 50°C und versetzt die trübe Lösung mit 350 ml Xylol, gefolgt von 30 g Prolit Rapid$^R$ (Filtrationshilfsmittel). Nach 15- minütigem Rühren filtriert man die Suspension und befreit das Filtrat am Rotationsverdampfer vom Lösungsmittel. Gemäss Zinnanalyse des verbleibenden flüssigen Chlorhydrinethers enthält dieser nur noch ca. 13 % der total mit dem Katalysator eingesetzten Zinnmenge, d.h. 87 % des Katalysators können mittels Filtration abgetrennt werden.

Den flüssigen Chlorhydrinether (589 g) erwärmt man auf 55°C und tropft bei dieser Temperatur unter gutem Rühren innert 30 Minuten 336 g einer 50 %-igen wässrigen Natriumhydroxidlösung (4,2 Mol) zu. Man rührt 2,5 Stunden bei 50-60°C, kühlt auf Raumtemperatur und filtriert die Suspension, wobei man mit Xylol nachwäscht. Die organische Phase des zweiphasigen, klaren Filtrats wird über Magnesiumsulfat getrocknet und filtriert, und das Filtrat wird am Rotationsverdampfer im Vakuum vom Lösungsmittel befreit (Badtemperatur 50°C). Man erhält 343 g (85% d. Th.) farblosen Butan-1,4-dioldiglycidylether der folgenden Produktqualität: Epoxidgruppengehalt: 7,81 Val/kg; totaler Chlorgehalt: 5,5 %; hydrolysierbarer Chlorgehalt: 131 ppm.

Beispiel 2 (Bistrimethylolpropan-tetraglycidylether):

Ein 1,5 Liter Reationsgefäss gemäss Beispiel 1 wird mit 250,13 g (1 Mol) Bistrimethylolpropan [der Firma Perstorp Chemicals;

$CH_3CH_2C(CH_2OH)_2$-$CH_2OCH_2C(CH_2OH)_2$-$CH_2CH_3$] beladen, und durch Erwärmen auf 120°C wird eine Schmelze erzeugt, die durch Anlegen eines Vakuum von ca. 30 mbar während 15 Minuten von eventuell vorhandenem Wasser befreit wird. Man fügt 3,13 g (0,02 Mol) fein pulverisiertes Zinndifluorid zu und dosiert 407 g (4,4 Mol) Epichlorhydrin mit einer Geschwindigkeit von 3 ml pro Minute unter gutem Rühren derart zu, dass die Innentemperatur zwischen 120 und 125°C bleibt. Die Reaktion ist anfänglich exotherm, später muss mit einem Oelbad (Badtemperatur 130°C) geheizt werden. Nach der Zudosierung werden nochmals 1,57 g (0,01 Mol) Zinndifluorid zugefügt und 6 Stunden bei 120-125°C gerührt. Im Wasserstrahlvakuum wird überschüssiges Epichlorhydrin aus dem Reaktor destilliert (Destillatmenge 52,75 g) und 200 g Isobutylmethylketon sowie 20 g Prolit Rapid$^R$ zum Reaktionsgemisch zugefügt und 30 Min. bei Raumtemperatur, dann unter Eisbadkühlung, gerührt. Man filtriert und wäscht den Rückstand mit 100 g Isobutylmethylketon. Das Filtrat (874 g) enthält gemäss Zinnanalyse ca. 1,75 g Zinn, d.h. 50% des eingesetzten Katalysators sind durch Filtration abtrennbar.

Das Filtrat (Epichlorhydrinether) erwärmt man auf 50-60°C und tropft bei dieser Temperatur 340 g (4,25 Mol) einer 50 %-igen wässrigen Natriumhydroxidlösung innert 1 Stunde unter gutem Rühren zu. Man lässt 2,5 Stunden bei 55-60°C nachreagieren, filtriert die Suspension, trennt das zweiphasige Filtrat im Scheidetrichter und extrahiert die wässrige Phase nochmals mit 250 g Isobutylmethylketon.

Die vereinigten organischen Phasen neutralisiert man durch Ausschütteln mit 10 %-iger Kaliumdihydrogenphosphatlösung, trocknet über Magnesiumsulfat, filtriert und entfernt das Lösungsmittel am Rotationsverdampfer. Das Rohprodukt (457 g) wird im Vakuum (< 2000 Pa bzw. 20 mbar) getrocknet. Man erhält 428,32 g (90,3 %, bezogen auf Bistrimethylolpropan) farblosen, flüssigen Bistrimethylolpropan-tetraglycidylether mit folgenden Analysenwerten:

Epoxidgruppengehalt: 6,35 Val/kg (75 % d.Th.); totaler Chlorgehalt: 4,10 %; hydrolysierbarer Chlorgehalt: 7000 ppm; Viskosität bei 25°C: 650 mPa.s; mittleres Molekulargewicht Mn (Gelpermeationschromatographie GPC): 532 (Styroleichung).

Beispiel 3 (Trimethylolpropan-triglycidylether):

Gemäss dem Verfahren von Beispiel 2 werden 134,18 g (1 Mol) Trimethylolpropan der Formel $CH_3CH_2C(CH_2OH)_3$, 3,13 g (0,03 Mol) Zinndifluorid, 305,25 g (3,3 Mol) Epichlorhydrin und 252 g 50 %-iger Natronlauge (3,15 Mol) umgesetzt. Durch Filtration auf der Chlorhydrinstufe können 60 % der eingesetzten Katalysatormenge abgetrennt , werden. Man gewinnt 281,7 g farblosen, flüssigen Trimethylolpropan-triglycidylether mit folgenden Analysenwerten:

Epoxidgruppengehalt: 6,96 Val/kg; Viskosität bei 25 °C: 140 mPa.s; totaler Chlorgehalt: 7,8 %; hydrolysierbarer Chlorgehalt: 2,3 %; mittleres Molekulargewicht Mn (GPC): 398.

Beispiel 4 (Sorbitol-glycidylether):

In einem 750 ml Reaktionsgefäss, versehen mit Rückflusskühler, Thermometer, Rührer und Dosiervorrichtung für Epichlorhydrin, werden 72,9 g (0,4 Mol) Sorbit und 1,25 g (0,008 Mol) Zinndifluorid in 88,5 g (0,957 Mol) Epichlorhydrin vorgelegt und auf 110°C Innentemperatur aufgeheizt. Mit einer Dosiergeschwindigkeit von 1,5 ml/Min. gibt man 132,16 g (1,429 Mol) Epichlorhydrin unter Rühren dazu, wobei die Temperatur zwischen 110-115°C gehalten wird. Man rührt weitere 8 Stunden bei 125-130°C. Im Wasserstrahlvakuum wird das überschüssige Epichlorhydrin abdestilliert, und der Rückstand wird mit 441 g Methylisobutylketon verdünnt. Bei 50°C werden 142,4 g einer 50 %-igen Natriumhydroxidlösung (1,78 Mol) unter gutem Rühren innert 1 Stunde zugetropft und weitere 2 Stunden bei dieser Temperatur nachreagiert. Das Reaktionsgemisch wird durch Zugabe von Trockeneis neutralisiert und filtriert, und die organische Phase wird über Magnesiumsulfat getrocknet. Nach dem Entfemen des Lösungsmittels am Rotationsverdampfer und Trocknen im Vakuum (2 Stunden/60°C) verbleiben 155,2 g Sorbitol-glycidylether mit folgenden Analysenwerten:
Epoxidgruppengehalt: 5,5 Val/kg; totaler Chlorgehalt: 15,56 %; hydrolysierbarer Chlorgehalt: 1,09 %; Viskosität bei 40°C: 2460 mPa.s; mittleres Molekulargewicht Mn (GPC): 658.

Beispiel 5 (Tricyclo-[5.2.1.0$^{2,6}$]decan-3(4),8(9)-dimethanol-diglycidylether):

In einem 750 ml Reaktionskolben, versehen mit Rückflusskühler, Thermometer, Rührer und Dosiervorrichtung für Epichlorhydrin, werden 196,29 g (1,0 Mol) Tricyclo-[5.2.1.0$^{2,6}$]-decan-3(4),8(9)-dimethanol (TCD-Alkohol DM; Produkt der Firma Hoechst AG) und 3,13 g (0,02 Mol) Zinndifluorid auf 120-130°C erwärmt und bei dieser Temperatur unter Rühren 194,3 g (2,1 Mol) Epichlorhydrin mit 2,5 ml/Min.
Geschwindigkeit zudosiert. Man rührt 12 Stunden bei 125 °C und destilliert im Vakuum noch vorhandenes Epichlorhydrin ab. Zum Rückstand gibt man bei 35 °C innert 1 Stunde 160 g einer 50 %-igen Natriumhydroxidlösung (2,0 Mol) unter gutem Rühren zu und lässt 2 Stunden bei 30 °C nachreagieren. Man filtriert die gebildete Suspension und verdünnt das Filtrat mit 500 ml Toluol. Die organische Phase schüttelt man mit 10 %-iger wässriger Kaliumdihydrogenphosphatlösung aus und trocknet über Magnesiumsulfat. Nach dem . Entfernen des Lösungsmittels am Rotationsverdampfer und Trocknen im Vakuum bei 100°C verbleiben 296,3 g (96 % d. Th. bezogen auf den Alkohol)
Tricyclo-[5.2.1.0$^{2,6}$]-decan-3(4),8(9)-dimethanol-diglycidylether mit folgenden Analysenwerten:
Epoxidgruppengehalt: 4,67 Val/kg; Viskosität bei 25°C: 500 mPa.s; totaler Chlorgehalt: 5,46 %; hydrolysierbarer Chlorgehalt: 2,07 %; mittleres Molekulargewicht Mn (GPC): 269.

Beispiel 6 (1-Hexanolglycidylether):

In ein Reaktionsgefäss, versehen mit Rührer, Rückflusskühler und Thermometer, gibt man 102,18 g (1,0 Mol) 1-Hexanol und 3,14 g (0,02 Mol) pulverisiertes Zinndifluorid und erwärmt auf 115°C Innentemperatur. Dann werden unter gutem Rühren 86,26 ml (1,1 Mol) Epichlorhydrin innert einer 1 Stunde zudosiert. Man lässt 8 Stunden nachreagieren, wobei die Temperatur langsam auf ungefähr 125°C ansteigt. Man kühlt auf 55°C ab und tropft bei dieser Temperatur unter gutem Rühren innert 30 Min. 88 g (1,1 Mol) einer 50 %-igen wässrigen Natriumhydroxidlösung zu. Man rührt 6 Stunden bei 55°C, kühlt auf Raumtemperatur, filtriert die Suspension und wäscht mit Essigsäureethylester nach. Die Phasen werden getrennt, und die wässrige Phase wird zweimal mit 100 ml Essigsäureethylester extrahiert. Die vereinigten organischen Phasen werden mit $CO_2$ neutralisiert, über Magnesiumsulfat getrocknet und filtriert, und das Filtrat wird am Rotationsverdampfer im Vakuum vom Lösungsmittel befreit (Badtemperatur 50°C). Man erhält 142,7 g (90,2 % d. Th.) farblosen 1-Hexanolglycidylether der folgenden Produktqualität: Epoxidgruppengehalt: 5,5 Val/kg (87 % d. Th.); totaler Chlorgehalt: 3,2 %; hydrolysierbarer Chlorgehalt: 0,21 %.

Beispiel 7 (Isooctanolglycidylether, Isomerengemisch):

In ein Reaktionsgefäss, versehen mit Rührer, Rückflusskühler, Tropftrichter und Thermometer, gibt man 130,23 g (1.0 Mol) Isooctanol (Isomerengemisch), 3,14 g (0,02 Mol) pulverisiertes Zinndifluorid und 5,28 g (0,02 Mol) 18-Crown-6 (Kronenether) und erwärmt auf 125°C Innentemperatur. Dann werden unter gutem Rühren 86,26 ml (1,1 Mol) Epichlorhydrin innert 30 Min. zudosiert. Man lässt 20 Stunden nachreagieren, wobei die Temperatur auf ungefähr 130°C ansteigt. Man kühlt auf 55°C ab und tropft bei dieser Temperatur unter gutem Rühren innert 30 Min. 88 g (1,1 Mol) einer 50 %-igen Natriumhydroxidlösung zu. Man rührt 6 Stunden bei 55-60°C, kühlt auf Raumtemperatur, filtriert die Suspension und wäscht mit Essigsäureethylester nach. Die Phasen werden getrennt, und die wässrige Phase wird zweimal mit 100 ml Essigsäureethylester extrahiert. Die vereinigten organischen Phasen werden mit möglichst wenig 10 %-iger $KH_2PO_4$-Lösung und dann mit Wasser neutral gewaschen, über Magnesiumsulfat getrocknet und filtriert, und das Filtrat wird am Rotationsverdampfer im Vakuum vom Lösungsmittel befreit (Badtemperatur 50°C).

Man erhält 180,7 g (97 % d. Th.) farblosen Isooctanolglycidylether (Isomerengemisch) der folgenden Produktqualität: Epoxidgruppengehalt: 4,71 Val/kg (88 % d. Th.); totaler Chlorgehalt: 2,7 %; hydrolysierbarer Chlorgehalt: 122 ppm.

Beispiel 8 (Isooctanolglycidylether, Isomerengemisch)

In ein Reaktionsgefäss, versehen mit Rührer, Rückflusskühler,Tropftrichter und Thermometer, gibt man 130,23 g (1,0 Mol) Isooctanol (Isomerengemisch), 4,51 g (0,02 Mol) $SnCl_2.2H_2O$ und 5,28 g (0,02 Mol) 18-Crown-6 und erwärmt auf 130°C Innentemperatur. Dann werden unter gutem Rühren 86,26 ml (1,1 Mol) Epichlorhydrin innert 30 Min. zudosiert. Man lässt 20 Stunden nachreagieren, wobei die Temperatur auf ungefähr 135°C ansteigt. Man kühlt auf 55°C ab und tropft bei dieser Temperatur unter gutem Rühren innert 30 Min. 88 g (1,1 Mol) einer 50 %-igen Natriumhydroxidlösung zu. Man rührt 5 Stunden bei 55-60°C, kühlt auf Raumtemperatur, filtriert die Suspension und wäscht mit Essigsäureethylester nach. Die Phasen werden getrennt, und die wässrige Phase wird zweimal mit 100 ml Essigsäureethylester extrahiert. Die vereinigten organischen Phasen werden mit möglichst wenig 10 %-iger $KH_2PO_4$-Lösung und dann mit Wasser neutral gewaschen, über Magnesiumsulfat getrocknet und filtriert, und das Filtrat wird am Rotationsverdampfer im Vakuum vom Lösungsmittel befreit (Badtemperatur 50°C).

Man erhält 167,4 g (90% d.Th.) leicht gelben Isooctanolglycidylether (Isomerengemisch) der folgenden Produktqualität: Epoxidgruppengehalt: 3,93 Val/kg (73%); totaler Chlorgehalt: 2,44 %; hydrolysierbarer Chlorgehalt: 232 ppm.

Beispiel 9 (Isooctanolglycidylether, Isomerengemisch):

In ein Reaktionsgefäss der in Beispiel 8 beschriebener Art gibt man 130,23 g (1,0 Mol) Isooctanol (Isomerengemisc)h, 5,57 g (0,02 Mol) pulverisiertes Zinndibromid und 5,28 g (0,02 Mol) 18-Crown-6 und erwärmt auf 130°C Innentemperatur. Dann werden unter gutem Rühren 86,26 ml (1,1 Mol) Epichlorhydrin innert 30 Min. zudosiert. Man lässt 12 Stunden nachreagieren, wöbei die Temperatur auf ungefähr 135°C ansteigt. Man kühlt auf 55°C ab und tropft bei dieser Temperatur unter gutem Rühren innert 30 Min. 88g (1,1 Mol) einer 50 %-igen Natriumhydroxidlösung zu. Man rührt 2,5 Stunden bei 55-60°C, kühlt auf Raumtemperatur, filtriert die Suspension und wäscht mit Essigsäureethylester nach. Die Phasen werden getrennt, und die wässrige Phase wird einmal mit 100 ml Essigsäureethylester extrahiert. Die vereinigten organischen Phasen werden mit möglichst wenig 10 %-iger $KH_2PO_4$-Lösung und dann mit Wasser neutral gewaschen, über Magnesiumsulfat getrocket und filtriert, und das Filtrat wird am Rotationsverdampfer im Vakuum vom Lösungsmittel befreit (Badtemperatur 50°C).

Man erhält 181,8 g (98% d. Th.) leicht gelben Isooctanolglycidylether (Isomerengemisch) der folgenden Produktqualität: Epoxidgruppengehalt: 3,69 Val/kg (69 % d.Th.); totaler Chlorgehalt: 3,31 %; hydrolysierbarer Chlorgehalt: 750 ppm.

Beispiel 10 (1,4-Cyclohexandiglycidylether):

In ein Reaktionsgefäss der in Beispiel 8 beschriebenen Art gibt man 116,16 g (1,0 Mol) 1,4-Cyclohexandiol und 3,14 g (0,02 Mol) pulverisiertes Zinndifluorid und erwärmt auf 130°C Innentemperatur, wobei das Edukt schmilzt. Dann werden unter gutem Rühren 152,12 ml (1,94 Mol) Epichlorhydrin innert 30 Min. zudosiert. Man lässt 5 Stunden nachreagieren, wobei die Temperatur auf ungefähr 135°C ansteigt. Man kühlt auf 55°C ab, fügt 250 ml Toluol zu und tropft bei dieser Temperatur unter gutem Rühren innert 30 Min. 155,2 g (1,94 Mol) einer 50 %-igen wässrigen Natriumhydroxidlösung zu. Man rührt 2,5 Stunden bei 55-60°C, kühlt auf Raumtemperatur, filtriert die Suspension und wäscht mit Toluol nach. Die Phasen werden getrennt, und die wässrige Phase wird fünfmal mit 50 ml Essigsäureethylester extrahiert. Die vereinigten organischen Phasen werden mit möglichst wenig 10 %-iger $KH_2PO_4$-Lösung und dann mit Wasser neutral gewaschen, über Magnesiumsulfat getrocknet und filtriert, und das Filtrat wird am Rotationsverdampfer im Vakuum vom Lösungsmittel befreit (Badtemperatur 60°C).

Man erhält 204,2 g (90% d. Th.) leicht gelben 1,4-Cyclohexandiglycidylether der folgenden Produktqualität: Epoxidgruppengehalt: 6,0 Val/kg (76 % d.Th.); totaler Chlorgehalt: 4,3 %; hydrolysierbarer Chlorgehalt: 750 ppm.

Beispiel 11 (Perhydrobisphenol-A-diglycidylether):

In ein Reaktionsgefäss der in Beispiel 8 beschriebener Art gibt man 240,4 g (1,0 Mol) Perhydrobisphenol-A, 3,14 g (0,02 Mol) pulverisiertes Zinndifluorid und 5,28 g (0,02 Mol) 18-Crown-6 und erwärmt auf 145°C Innentemperatur., wobei das Edukt schmilzt. Dann werden unter gutem Rühren 152,12 ml (1,94 Mol) Epichlorhydrin innert 30 Min. zudosiert. Man lässt 12 Stünden nachreagieren. Man kühlt auf 55°C ab, fügt 250 ml Toluol zu und tropft bei dieser Temperatur unter gutem Rühren innert 30 Min. 155,2 g (1,94 Mol) einer 50 %-igen wässrigen Natriumhydroxidlösung zu. Man rührt 4,5 Stunden bei 55-60°C, kühlt auf Raumtemperatur, filtriert die Suspension und wäscht mit Toluol nach. Die Phasen werden getrennt, und die wässrige Phase wird zweimal mit 100 ml Toluol extrahiert. Die vereinigten organischen Phasen werden mit möglichst wenig 10 %-iger $KH_2PO_4$-Lösung und dann mit Wasser neutral gewaschen, über Magnesiumsulfat getrocknet und filtriert, und das Filtrat wird am Rotationsverdampfer im Vakuum vom Lösungsmittel befreit (Badtemperatur 70°C).
Man erhält 330,8 g (94 % d. Th.) leicht gelben Perhydrobisphenol-A-diglycidylether der folgenden Prokuktqualität: Epoxidgruppengehalt: 4,31 Val/kg (76 % d.Th.); totaler Chlorgehalt: 2,1 %; hydrolysierbarer Chlorgehalt: 171 ppm.

## Patentansprüche

1. Verbessertes Verfahren zur Herstellung von Glycidylethern der Formel I

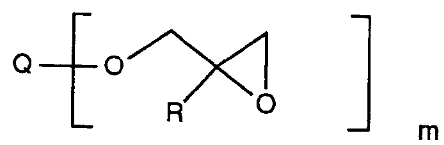

(I),

worin Q für einen m-wertigen aliphatischen, cycloaliphatischen oder araliphatischen Rest und R für -H oder $-CH_3$ stehen, und m eine ganze Zahl von 1 bis 10 ist, indem ein Alkohol der Formel $Q\text{-}(OH)_m$ mit m Mol eines Epihalohydrins der Formel II

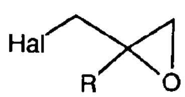

(II)

in Gegenwart eines Katalysators zum entspechenden Halohydrinether umgesetzt wird und der Halohydrinether mit einem Alkalimetallhydroxid zu einer Verbindung der Formel I dehydrohalogeniert wird, wobei Q, m und R die oben angegebene Bedeutung haben und Hal für Halogen steht, dadurch gekennzeichnet, dass man als Katalysator
   a) Zinndifluorid oder
   b) ein zweiwertiges Zinnhalogenid in Kombination mit einem Co-Katalysator verwendet.

2. Verfahren gemäss Anspruch 1, wonach man als Epihalohydrin Epibromhydrin oder Epichlorhydrin verwendet.

3. Verfahren gemäss Anspruch 2, wonach man Epichlorhydrin verwendet.

4. Verfahren gemäss Anspruch 1, wonach zwischen 0,8 und 1,3 Mol Epihalohydrin der Formel II pro Hydroxylgruppe gemäss der Formel $Q\text{-}(OH)_m$ eingesetzt werden.

5. Verfahren gemäss Anspruch 1, wonach als zweiwertiges Zinnhalogenid Zinndichlorid, Zinndibromid oder Zinndifluorid verwendet wird.

6. Verfahren gemäss Anspruch 1, wonach Zinndifluorid allein verwendet wird.

7. Verfahren gemäss Anspruch 1, wonach der Zinnhalogenid-Katalysator in einer Konzentration von 0,001 bis 0,5 Mol pro Mol Alkohol der Formel Q-$(OH)_m$ verwendet wird.

8. Verfahren gemäss Anspruch 1, wonach als Co-Katalysator ein Kronenether oder ein Poly(alkylenoxid)-dialkylether eingesetzt wird.

9. Verfahren gemäss Anspruch 1, wonach in der Formel I m eine ganze Zahl von 1 bis 6 ist.

10. Verfahren gemäss Anspruch 1, wonach Q einen gesättigten aliphatischen oder cycloaliphatischen Rest bedeutet.

11. Verfahren gemäss Anspruch 1, wonach Q als aliphatischer Rest ein gesättigtes Alkyl ist.

12. Verfahren gemäss Anspruch 1, wonach als Verbindungen der Formel Q-$(OH)_m$ primäre oder sekundäre monofunktionelle (m=1) oder mehrfunktionelle Alkohole, bei welchen m eine ganze Zahl von 2 bis 6 darstellt, verwendet werden.

13. Verfahren gemäss Anspruch 1, wonach in der Formel Q-$(OH)_m$ Q einen mehrfunktionellen Rest mit bis zu 30 C-Atomen darstellt, wobei m eine Zahl von 2 bis 6 ist.

14. Verfahren gemäss Anspruch 1, wonach die Dehydrohalogenierung des Halohydrinethers zum Glycidylether mit Hilfe von Natriumhydroxid durchgeführt wird.